# FASCICULE DE BREVET EUROPEEN

(11) **EP 0 426 552 B1**
(45) Date de publication et mention de la délivrance du brevet: **31.08.1994**
(21) Numéro de dépôt: 90403054.1
(22) Date de dépôt: 29.10.1990
(51) Int. Cl.: C12Q 1/28, C12Q 1/62

(54) **Composition de peroxydase stabilisée, utilisable pour des essais immuno-enzymatiques**
In Immunoassays verwendbare stabilisierte Peroxidase-Zusammensetzung
Composition of stabilized peroxidase, for use as immuno-enzymatic assays

(30) Priorité: 30.10.1989 FR 8914213
(43) Date de publication de la demande: 08.05.1991
(73) Titulaire: CIS BIO INTERNATIONAL, 91400 Saclay (FR)
(72) Inventeur: Dedieu, Alain, F-84000 Avignon (FR); Salard, Jean-Louis, F-30200 Bagnols Sur Ceze (FR)
(74) Mandataire: Des Termes, Monique

(56) Documents cités:
- EP-A- 0 197 447
- CHEMICAL ABSTRACTS, vol. 110, no. 21, 22 mai 1989, Columbus, OH (US); G.L. FOUREMAN et al., p. 248, no. 187538m#

## Description

La présente invention concerne une composition stabilisée d'une enzyme, utilisable en particulier dans des dosages immuno-enzymatiques.

Dans les dosages immuno-enzymatiques qui sont bases sur des réactions du type antigène-anticorps, on utilise un marqueur constitué par un composé conjugué entre, d'une part, un antigène, un haptène ou un anticorps et, d'autre part, une enzyme. Des dosages immuno-enzymatiques de ce type sont décrits par exemple dans J. Clin. Chem. Clin. Biochem., vol.18, 1980, pages 197-208.

Pour ces dosages, il est important de choisir une enzyme qui conduise à un compose conjugué ayant une haute activité spécifique permettant de réaliser des dosages à haute limite de détection, mais ayant par ailleurs, une bonne stabilité au stockage.

De nombreuses enzymes peuvent être utilisées dans ces dosages, mais certaines comme les peroxydases présentent l'inconvénient de former des composés conjugués relativement instables, en particulier à faible concentration. Parmi les peroxydases, la peroxydase de Raifort est largement employée dans les composés conjugués des trousses de dosage utilisées en analyse biologique.

Ces trousses sont commercialisées et leur réputation est basée sur la robustesse de leurs composants, en particulier celle de leur traceur enzymatique. Or, ces trousses sont souvent expédiées, puis stockées pendant des durées très variables, dans des lieux où la température est rarement contrôlée. De ce fait, les composés de la trousse subissent des chocs thermiques qui peuvent affecter leur stabilité. Aussi, si aucune précaution n'est prise, la stabilité annoncée de l'enzyme ne se maintient pas dans le composé conjugué qui perd rapidement son activité enzymatique.

Pour pallier cet inconvénient, on a envisagé d'ajouter au composant de la trousse contenant le composé conjugué une dose efficace d'un agent stabilisant l'activité de l'enzyme. Les agents stabilisants utilisés sont par exemple, l'acide 8-anilino-1-naphtalène sulfonique, la gentamicine, l'amikacine, la tobramycine et la 4-aminoantipyrine, comme il est décrit dans les documents US-A-4 252 896, US-A-4 504 579 et EP-A-0 070 992.

La présente invention a pour objet l'utilisation d'un autre agent stabilisant dans des compositions de ce type utilisables pour des dosages immuno-enzymatiques.

Selon l'invention, la composition comprend une peroxydase et une quantité efficace d'un agent stabilisant, et elle se caractérise en ce que l'agent stabilisant est l'acide urique.

Dans cette composition, la peroxydase peut être sous forme élémentaire, ou le plus fréquemment sous la forme de composé, en particulier de composé conjugué constitué d'une molécule de réactif immunologique choisi parmi les antigènes, les haptènes, les anticorps et leurs fragments, lié à la peroxydase.

La liaison peut être une liaison de covalence, mais il peut s'agir aussi d'une liaison réversible.

Dans cette composition, on suppose que la stabilité de l'enzyme est obtenue en retardant la dégradation du centre actif de l'enzyme.

En effet, la perte d'activité d'un composé conjugué ne provient généralement pas d'une rupture de la liaison enzyme-réactif immunologique (anticorps, antigène ou haptène) établie au cours de la synthèse du conjugué, mais plutôt de perturbations survenant au niveau du site enzymatique constitué d'un hème au centre duquel se trouve un atome de fer. Or, on a trouvé que l'acide urique permettait d'éviter les perturbations survenant au niveau de ce site enzymatique.

Les compositions de l'invention peuvent être sous la forme de solution, par exemple de solutions de la peroxydase ou du composé conjugué dans un milieu liquide ou, sous la forme de poudres sèches obtenues par lyophilisation d'une solution de peroxydase ou d'un composé conjugué de peroxydase dans un milieu liquide.

Dans les deux cas, la concentration en agent stabilisant de la solution finale ou de la solution avant lyophilisation peut aller jusqu'à la limite de solubilité de l'acide urique dans le milieu liquide. Elle est de préférence de 0,1 mmol/l à 2 mmol/l, par exemple de 0,5 mmol/l à 2 mmol/l.

Les milieux liquides peuvent être de différents types, mais on utilise le plus souvent des solutions tampons contenant une protéïne du sérum, par exemple un tampon phosphate contenant du sérum de boeuf.

La concentration en peroxydase ou en composé conjugué du milieu liquide peut varier dans une large gamme, par exemple de 1 ng/ml à 10 µg/ml.

Dans le composé conjugué, le réactif immunologique peut être un antigène ou un haptène choisi par exemple parmi les médicaments tels que les analgésiques, les antibiotiques, les anticonvulsants, les agents antidiabétiques, les agents antihypertensions, les agents antinéoplasiques, les barbiturates, les dépresseurs cardiaques, les glycosides cardiaques, les hallucinogènes, les insecticides, les agents relaxant des muscles, les stéroïdes, les stimulants, les alcaloïdes du tabac et les tranquillisants. Ce réactif peut aussi être constitué par une protéïne du plasma ou une hormone, par exemple un polypeptide, un stéroïde ou d'autres molécules plus petites.

A titre d'exemple, les antigènes peuvent être des protéïnes du sérum telles que les IgG, IgE, l'haptoglobine, l'alpha-2H-globuline, l'alpha-FP, et le CEA (antigène carcino embryonnaire) ; les hormones peuvent être HCG, HPL, TSH, l'insuline, les oestrogènes, la progestérone, le cortisol et la thyroxine ; et les médicaments peuvent être des barbiturates, des opiates, la méthadone, des amphétamines et la digoxine.

Le réactif immunologique peut encore être constitué par des anticorps des antigènes et haptènes décrits ci-dessus, ou des anticorps, de certains pathogènes, en particulier des anticorps monoclonaux ou leurs fragments.

Les peroxydases utilisables dans la composition de l'invention peuvent être de différents types et de différentes origines. On peut utiliser par exemple les peroxydases provenant des plantes, telles que la peroxydase de Raifort, les peroxydases provenant d'animaux vertébrés telles que les lactopéroxydases, les peroxydases provenant de micro-organismes telle la peroxydase de Pseudomonas.

Selon l'invention, on peut aussi ajouter à la composition de peroxydase d'autres constituants, en particulier des protéïnes sériques, par exemple du sérum de foetus de veau, du sérum de lapin, du sérum de porc, des tampons tels que le tampon tris-HCl, et éventuellement d'autres agents stabilisants.

La composition de peroxydase stabilisée de l'invention peut être sous la forme d'une solution ou d'un produit lyophilisé comme on l'a vu précédemment. On peut aussi l'utiliser sur un support solide tel qu'un support absorbant. Le support absorbant peut être préparé à partir de papier, de structure poreuse particulaire, de film polymère poreux, de cellulose, de fibres de verre, de tissus tissés et non tissés synthétiques et non synthétiques.

D'autres caractéristiques et avantages de l'invention apparaîtront mieux à la lecture des exemples suivants donnés bien entendu à titre illustratif et non limitatif.

### Exemple 1 : Composition stabilisée d'un composé conjugué anti-ACE-peroxydase de Raifort

On prépare cette composition de la façon suivante.

On traite thermiquement à 55°C pendant 1 heure du sérum de boeuf disponible commercialement pour éliminer toute activité peroxydative endogène. On filtre ensuite le sérum pour éliminer tout précipité et on dilue le filtrat avec 0,1 mol/litre de tampon phosphate (pH 7,0) pour obtenir une concentration finale en sérum de boeuf de 10 %. On introduit cette composition dans 4 séries de flacons différents. Dans la première série de flacons, on ajoute 2 mmol/litre d'acide urique ; dans la deuxième série de flacons, on ajoute 1 mmol/litre d'acide urique, dans la troisième série de flacons, on ajoute 0,5 mmol/litre d'acide urique. On n'ajoute pas d'acide urique dans la quatrième série de flacons.

On ajoute ensuite dans les flacons de toutes les séries un compose conjugué de peroxydase de Raifort et d'anti corps anti-ACE de façon à obtenir dans chaque série des flacons ayant des concentrations en composé conjugué de 100 ng/ml. On filtre les solutions à travers des filtres stériles et on les réintroduit dans des flacons. On fait subir ou non à ces préparations des contraintes thermiques qui simulent leur vieillissement.

On utilise ensuite ces solutions de conjugué dans un test au cours duquel et de façon simultanée, dans un tube à essai, un antigène (100 µl) à forte ou à faible concentration (standard haut ou standard bas) est mis à incuber vis-à-vis d'anticorps anti CEA fixés sur une phase solide et de solution de conjugué (200 µl) anti CEA déjà préparée.

L'incubation dure 2 heures à 37°C. Après lavage des tubes, on révèle pendant 30 minutes un mélange substrat contenant un tampon citrate, du peroxyde d'hydrogène et de l'o-phénylène diamine.

Après cette incubation, on ajoute 1 ml d'acide oxalique 1M dans chaque tube et on peut mesurer l'activité enzymatique, spectrophotométriquement sur un analyseur bichromatique à 490 nm.

L'activité enzymatique est exprimée en pourcentage de l'activité enzymatique d'un flacon identique conservé à l'obscurité et à 4°C, pendant la même durée.

Les résultats obtenus sont donnés dans le tableau 1 qui suit.

Au vu de ces résultats, on constate que l'acide urique est très efficace pour maintenir l'activité enzymatique du composé, bien que celui-ci ait été stocké dans des conditions très défavorables.

### Exemple 2 : Composition stabilisée d'un composé conjugué anti-ACE-peroxydase de Raifort.

On suit le même mode opératoire que dans l'exemple 1 pour préparer une composition de conjugué anti-ACE-peroxydase de Raifort et déterminer l'activité enzymatique des flacons contenant 1 mmole/litre d'acide urique après stockage pendant 4 jours à 37°C, et après stockage pendant 4 jours à 37°C puis 2 jours à -20°C.

Les résultats obtenus sont donnés dans le tableau 2 qui suit.

### Exemple 3 : Composition stabilisée d'un composé conjugué anti-TSH-peroxydase de Raifort

Dans cet exemple, on prépare comme dans l'exemple 1 des flacons contenant un tampon phosphate et du sérum de boeuf et on ajoute dans certains flacons 1 mmole/litre d'acide urique et dans tous les flacons un composé conjugué de peroxydase de Raifort et d'anti corps anti-TSH de façon à obtenir une concentration de 30 ng/ml.

On détermine l'activité enzymatique des flacons comme dans l'exemple 1 après un stockage pendant 14 jours soit 5 jours à 37°C et 9 jours à -20°C.

Les résultats obtenus sont donnés dans le tableau 3 qui suit.

**TABLEAU 1**

| conjugué (anti ACE) : influence de la concentration en acide urique | | | | |
|---|---|---|---|---|
| Conjugué conditionné dans : | | Activité enzymatique (en %) | | |
| | | Au jour 0 | Après 2j à 37°C | Après 2j à 37°C puis 2j à - 20°C |
| Tampon seul | Standard bas | 100 | 87 | 30 |
| | Standard haut | 100 | 89 | 40 |
| Tampon + acide urique 2mM | Standard bas | 100 | 100 | 77 |
| | Standard haut | 100 | 95 | 92 |
| Tampon + acide urique 1mM | Standard bas | 100 | 100 | 70 |
| | Standard haut | 100 | 94 | 83 |
| Tampon + acide urique 0,5mM | Standard bas | 100 | 94 | 77 |
| | Standard haut | 100 | 88 | 79 |

**TABLEAU 2**

| conjugué (anti ACE) - rôle de l'acide urique | | | | |
|---|---|---|---|---|
| Conjugué conditionné dans : | | Activité enzymatique du conjugué (en %) | | |
| | | Au jour 0 | Après 4 j à 37°C | Après 4 j à 37°C + 2j à -20°C |
| Tampon seul | Standard bas | 100 | 42 | 6 |
| | Standard haut | 100 | 54 | 12,5 |
| Tampon + acide urique 1mM | Standard bas | 100 | 86 | 77 |
| | Standard haut | 100 | 94 | 83 |

**TABLEAU 3**

| conjugué anti TSH - rôle de l'acide urique | | | | |
|---|---|---|---|---|
| Conjugué conditionné dans : | | Activité enzymatique du conjugué (en %) | | |
| | | Au jour 0 | Après 5j à 37°C | Après 14j dont 5j à 37°C et 9j à -20°C |
| Tampon seul | Standard bas | 100 | 100 | 41 |
| | Standard haut | 100 | 94 | 50 |
| Tampon + acide urique 1mM | Standard bas | 100 | 99 | 90 |
| | Standard haut | 100 | 99 | 86 |

## Revendications

1. Composition comprenant une peroxydase et une quantité efficace d'un agent stabilisant, caractérisée en ce que l'agent stabilisant est l'acide urique.

2. Composition selon la revendication 1, caractérisée en ce que la peroxydase est sous la forme d'un composé conjugué constitué d'une molécule choisie parmi les antigènes, les haptènes, les anticorps et leurs fragments, liée à la peroxydase.

3. Composition selon la revendication 2, caractérisée en ce que le composé conjugué est dans un milieu liquide et en ce que la concentration en agent stabilisant du milieu liquide est de 0,1 mmole/litre à 2 mmoles/litre.

4. Composition selon la revendication 3, caractérisée en ce que le milieu est une solution tampon phosphate contenant du sérum de boeuf.

5. Composition selon l'une quelconque des revendications 2 à 4, caractérisée en ce que la molécule est un anticorps de l'antigène carcino-embryonnaire.

6. Composition selon l'une quelconque des revendications 2 à 4, caractérisée en ce que la molécule est un anticorps de la TSH (hormone de stimulation de la thyroïde).

7. Composition selon l'une quelconque des revendications 1 à 6, caractérisée en ce que la peroxydase est la peroxydase de Raifort.

## Claims

1. Composition comprising a peroxidase and an effective amount of a stabilizing agent, characterized in that the stabilizing agent is uric acid.

2. Composition according to Claim 1, characterized in that the peroxidase is in the form of a conjugated compound consisting of a molecule chosen from antigens, haptens and antibodies and fragments thereof, linked to the peroxidase.

3. Composition according to Claim 2, characterized in that the conjugated compound is in a liquid medium, and in that the concentration of stabilizing agent in the liquid medium is from 0.1 mmol/litre to 2 mmol/litre.

4. Composition according to Claim 3, characterized in that the medium is a phosphate buffer solution containing bovine serum.

5. Composition according to any one of Claims 2 to 4, characterized in that the molecule is an antibody to carcinoembryonic antigen.

6. Composition according to any one of Claims 2 to 4, characterized in that the molecule is an antibody to TSH (thyroid-stimulating hormone).

7. Composition according to any one of Claims 1 to 6, characterized in that the peroxidase is horseradish peroxidase.

## Patentansprüche

1. Zusammensetzung, umfassend eine Peroxidase und eine wirksame Menge eines Stabilisierungsmittels, **dadurch gekennzeichnet**, daß das Stabilisierungsmittel Harnsäure ist.

2. Zusammensetzung nach Anspruch 1, **dadurch gekennzeichnet**, daß die Peroxidase in Form einer konjugierten Verbindung vorliegt, die aus einem Molekül, ausgewählt unter Antigenen, Haptenen, Antikörpern und ihren Fragmenten, welches an die Peroxidase gebunden ist, aufgebaut ist.

3. Zusammensetzung nach Anspruch 2, **dadurch gekennzeichnet**, daß die konjugierte Verbindung in einem flüssigen Medium vorliegt, und daß die Konzentration des Stabilisierungsmittels in dem flüssigen Medium 0,1 mmol/Liter bis 2 mmol/Liter beträgt.

4. Zusammensetzung nach Anspruch 3, **dadurch gekennzeichnet**, daß das Medium eine Phosphatpufferlösung, die Rinderserum enthält, ist.

5. Zusammensetzung nach einem der Ansprüche 2 bis 4, **dadurch gekennzeichnet**, daß das Molekül ein Antikörper des karzinoembryogenen Antigens ist.

6. Zusammensetzung nach einem der Ansprüche 2 bis 4, **dadurch gekennzeichnet**, daß das Molekül ein Antikörper des TSH (thyreotropen Hormons) ist.

7. Zusammensetzung nach einem der Ansprüche 1 bis 6, **dadurch gekennzeichnet**, daß die Peroxidase die Meerrettich-Peroxidase ist.
